# EUROPÄISCHE PATENTSCHRIFT

(11) **EP 0 247 466 B1**
(45) Veröffentlichungstag und Bekanntmachung des Hinweises auf die Patenterteilung: **24.11.1993**
(21) Anmeldenummer: 87107124.7
(22) Anmeldetag: 16.05.1987
(51) Int. Cl.: C07C 69/92, C07C 69/76, C07C 255/00, C09K 19/30

(54) **Flüssigkristalline Derivate von Phenylbenzoat**
Liquid-crystal derivatives of phenyl benzoate
Dérivés de benzoates phényliques sous forme de cristaux liquides

(30) Priorität: 22.05.1986 CH 2066/86; 16.03.1987 CH 972/87
(43) Veröffentlichungstag der Anmeldung: 02.12.1987
(73) Patentinhaber: F. HOFFMANN-LA ROCHE AG, 4002 Basel (CH)
(72) Erfinder: Buchecker, Richard, Dr., CH-40503 Basel (CH); Fromm, Hans-Jürgen, Dr., D-7850 Lörrach (DE); Kelly, Stephen, Dr., CH-4303 Kaiseraugst (CH); Schadt, Martin, Dr., CH-4411 Seltisberg (CH)
(74) Vertreter: Cottong, Norbert A.

(56) Entgegenhaltungen:
- EP-A- 0 035 155
- EP-A- 0 103 681
- PATENT ABSTRACTS OF JAPAN, unexamined applications, C Sektion, Band 7, Nr. 252, 9. November 1983 THE PATENT OFFICE JAPANESE GOVERNMENT Seite 146 C 194
- PATENT ABSTRACTS OF JAPAN, unexamined applications, C Sektion, Band 9, Nr. 7, 24. JUnner 1985 THE PATENT OFFICE JAPANESE GOVERNMENT Seite 148 C 262

## Beschreibung

Die vorliegende Erfindung betrifft neue flüssigkristalline Ester, deren Herstellung, flüssigkristalline Gemische, die solche Ester enthalten, sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann gut bekannt und können auf verschiedenen Effekten beruhen, wie beispielsweise der dynamischen Streuung, der Deformation aufgerichteter Phasen (DAP-Zelle), dem Schadt-Helfrich--Effekt (TN-Zelle [twisted-nematic] und STN-Zelle [super twisted-nematic]), dem Gast/Wirt-Effekt (Guest/Host-Zelle), einem cholesterisch-nematischen Phasenübergang (Phase--Change-Zelle) oder dem SBE-Effekt (super birefringence effect).

Cholesterische Flüssigkristalle finden beispielsweise in der oben erwähnten Phase-Change-Zelle Verwendung. Ferner können cholesterische Zusätze oder andere chirale Substanzen bei geeigneter Wahl der Konzentration auch zur Verbesserung der elektro-optischen Eigenschaften von Flüssigkristallen für Drehzellenanzeigen eingesetzt werden. Nematische Flüssigkristallkomponenten können in allen, oben genannten Anwendungen eingesetzt werden.

Die genannten Anzeigevorrichtungen besitzen im allgemeinen Ansprechzeiten in der Grössenordnung von mehreren Millisekunden oder höher. Zur Verbesserung der Ansprechzeiten der Anzeigevorrichtungen wurden in letzter Zeit auch Flüssigkristalle mit ferroelektrischen Eigenschaften eingesetzt. Bei dieser Anwendung werden chirale smektische Phasen, beispielsweise smektisch C, F oder I Phasen, meist smektisch C Phasen verwendet. Bisher sind jedoch relativ wenige derartige Flüssigkristalline bekannt und deren Stabilität ist oft ungenügend.

Damit Flüssigkristalle zur Anwendung in Anzeigevorrichtungen geeignet sind, sollten sie eine gute chemische und thermische Stabilität und eine gute Stabilität gegenüber elektrischen Feldern und elektromagnetischer Strahlung aufweisen. Ferner sollten sie farblos sein und niedere Viskositäten aufweisen und in Anzeigevorrichtungen kurze Ansprechzeiten und einen hohen Kontrast ergeben. Weiterhin sollten die Flüssigkristalle bei üblichen Betriebstemperaturen eine geeignete Mesophase, beispielsweise eine cholesterische oder eine geeignete chirale smektische Phase aufweisen. Da Flüssigkristalle üblicherweise als Mischungen zur Anwendung gelangen, ist es zudem wichtig, dass die Komponenten untereinander gut mischbar sind. Weitere Eigenschaften, wie beispielsweise die Schwellenspannung, die dielektrische Anisotropie und die optische Anisotropie, müssen je nach verwendetem Zellentyp unterschiedliche Bedingungen erfüllen. Flüssigkristalle mit ferroelektrischen Eigenschaften besitzen vorzugsweise eine negative dielektrische Anisotropie oder einen kleinen absoluten Wert der dielektrischen Anisotropie.

Gegenstand der Erfindung sind die tricyclischen und tetracyclischen Ester der allgemeinen Formel 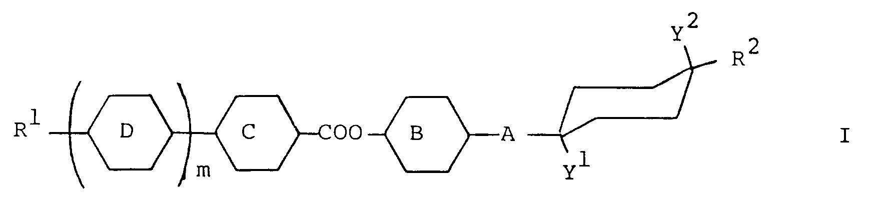
worin m für die Zahl 0 oder 1 steht; A eine einfache Kovalenzbindung, -CH₂-CH₂-, -OCH₂ -, -COO- oder -OOC- bedeutet; die Ringe B, C und D gegebenenfalls mit Cyano, Halogen oder Niederalkyl substituiertes 1,4--Phenylen bezeichnen; Y¹ und Y² Wasserstoff bedeuten oder einer der Substituenten Y¹ und Y² auch Cyano bedeutet; und R¹ und R² unabhängig voneinander gegebenenfalls halogensubstituiertes C₁-C₁₈-Alkyl oder gegebenenfalls halogensubstituiertes C₂-C₁₈-Alkenyl darstellen, in welchen gegebenenfalls eine oder zwei nicht benachbarte CH₂-Gruppen durch Sauerstoff ersetzt sind; mit der Massgabe, dass mindestens einer der Reste R¹ und R² ein chirales Kohlenstoffatom und/oder eine C-C-Doppelbindung aufweist, wenn A für -COO- steht, und mindestens einer der Reste R¹ und R² eine C-C-Doppelbindung aufweist, wenn A für eine einfache Kovalenzbindung steht.

Die erfindungsgemässen Verbindungen sind geeignete Komponenten für ferroelektrische Flüssigkristalle und besitzen grösstenteils selbst eine chirale smektische Phase. Sie eignen sich aber grundsätzlich auch für cholesterische oder nematische Gemische. Sie sind optisch aktiv oder optisch inaktiv und besitzen die oben angegebenen erforderlichen Eigenschaften.

Der obige Ausdruck "Halogen" umfasst Fluor, Chlor oder Brom.

Der Ausdruck "Niederalkyl" umfasst Alkylgruppen mit 1-5 Kohlenstoffatomen, wie Methyl, Aethyl, Propyl und Isopropyl, vorzugsweise Methyl.

Der Ausdruck "gegebenenfalls mit Cyano, Halogen oder Niederalkyl substituiertes 1,4-Phenylen" umfasst Gruppen wie 1,4-Phenylen, 2-Cyano-1,4-phenylen, 2,3-Dicyano-1,4-phenylen, 2-Fluor-1,4-phenylen, 2,3-Difluor-1,4-phenylen, 2-Chlor-1,4-phenylen, 2-Brom-1,4-phenylen, 2-Methyl-1,4--phenylen und dergleichen. Bevorzugt ist im allgemeinen 1,4-Phenylen. Durch Verwendung substituierter Gruppen können aber gewünschtenfalls die Umwandlungstemperaturen, die Löslichkeit, die dielektrische Anisotropie und dergleichen modifiziert und höher geordnete Phasen unterdrückt werden.

Der Ausdruck "Alkyl oder Alkenyl, in welchen gegebenenfalls eine oder zwei nicht benachbarte CH₂-Gruppen durch Sauerstoff ersetzt sind" umfasst geradkettige und verzweigte Gruppen wie Alkyl, Alkoxy, Alkoxyalkyl, Alkoxyalkoxy, Alkenyl, Alkenyloxy, Alkoxyalkenyl, Alkenyloxyalkyl, Alkoxyalkenyloxy und dergleichen. Entsprechende halogensubstituierte Gruppen sind beispielsweise Halogenalkyl, Halogenalkoxy, Halogenalkenyl, Halogenalkenyloxy und dergleichen.

Verbindungen der Formel I, worin Y¹ oder Y² Cyano bedeutet, besitzen eine negative Anisotropie der Dielektrizitätskonstanten. Im allgemeinen stehen jedoch Y¹ und Y² vorzugsweise für Wasserstoff. C-C-Doppelbindungen in R¹ und/oder R² führen vielfach zu einer Verbesserung der chiral smektischen Eigenschaften.

Eine bevorzugte Gruppe erfindungsgemässer Verbindungen sind diejenigen, worin Y¹ und Y² Wasserstoff bedeuten und Ring C und ein gegebenenfalls vorhandener Ring D für 1,4-Phenylen stehen. Ring B bezeichnet vorzugsweise 1,4--Phenylen, 2-Cyano-1,4-phenylen, 2,3-Dicyano-1,4-phenylen, 2-Halogeno-1,4-phenylen oder 2,3-Dihalogen-1,4-phenylene.

Eine weitere Gruppe bevorzugter Verbindungen der Formel I sind diejenigen, worin A -CH₂CH₂-, -OCH₂- oder -OOC-, insbesondere -CH₂CH₂- oder -OCH₂- bedeutet.

In obiger Formel I steht m vorzugsweise für die Zahl 0.

Ein weiterer bevorzugter Aspekt betrifft optisch aktive Verbindungen der Formel I, welche in R¹ und/oder R² ein chirales Kohlenstoffatom aufweisen.

Bevorzugte Gruppen R¹ bzw. R² mit chiralen Kohlenstoffatomen sind die Gruppen der allgemeinen Formeln 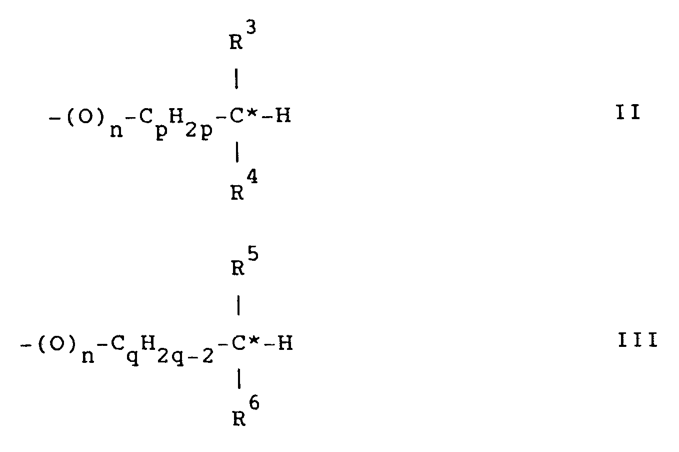
worin n, p und q ganze Zahlen bedeuten und n für 0 oder 1, p für 0-6 und q für 2-6 stehen; R³ Alkyl und R⁴ Halogen, Alkoxy, Alkenyl, Alkenyloxy oder von R³ verschiedenes Alkyl bedeuten; oder R³ Alkenyl und R⁴ Alkoxy bedeuten; R⁵ Alkyl und R⁶ Halogen, Alkoxy oder von R⁵ verschiedenes Alkyl bezeichnen; und C* das chirale Kohlenstoffatom bedeutet.

Besonders bevorzugt sind Gruppen der Formel II worin R³ Methyl und R⁴ von Methyl verschiedenes Alkyl, vorzugsweise Aethyl, bedeutet.

Im Falle optisch aktiver Verbindungen der Formel I besitzt vorzugsweise R¹ ein chirales Kohlenstoffatom. Besonders bevorzugt sind dabei diejenigen Verbindungen der Formel I, worin R¹ für eine Gruppe der Formel II oder III steht.

Bevorzugte Reste R² sind Alkyl, Alkenyl, Alkoxyalkenyl und Alkenyloxyalkyl. Falls R¹ ein chirales Kohlenstoffatom aufweist, steht R² vorzugsweise für einen geradkettigen Rest.

Besonders bevorzugt sind im allgemeinen diejenigen Verbindungen der Formel I, worin R¹ für eine chirale oder achirale Alkoxy oder Alkenyloxygruppe und R² für eine chirale oder achirale Alkyl- oder Alkenylgruppe steht. R² ist vorzugsweise achiral.

Vorzugsweise steht eine in R¹ und/oder R² gegebenenfalls vorhandene Doppelbindung in Stellung 1, 3 oder 4 (unter Einschluss allfällig vorhandener Sauerstoffatome) oder in endständiger Position der Seitenkette R¹ bzw. R², insbesondere in Stellung 4. Besonders bevorzugte ungesättigte Gruppen sind somit Gruppen wie 1E-Alkenyl, 3E-Alkenyl, 4-Alkenyl, 2E-Alkenyloxy, 3-Alkenyloxy, Alkoxy-3E-alkenyl, Alkoxy-4-alkenyl, Alkoxy-3-alkenyloxy, 5-Hexenyl, 4-Pentenyloxy, 6-Heptenyl, 5-Hexenyloxy, 7-Octenyl, 6-Heptenyloxy, 8-Nonenyl, 7-Octenyloxy, 9-Decenyl, 8-Nonenyloxy und dergleichen.

Die Reste R¹ und R² weisen zweckmässigerweise höchstens je 18 Kohlenstoffatome auf. Die Gruppe bzw. Gruppen mit chiralem Kohlenstoffatom besitzen vorzugsweise 4-18 und besonders bevorzugt 4-15 Kohlenstoffatome. Eine gegebenenfalls vorhandene Gruppe R¹ oder R² ohne chirales Kohlenstoffatom besitzt vorzugsweise höchstens 12 und besonders bevorzugt höchstens 7 Kohlenstoffatome. Für chiral smektische Anwendungen sind jedoch im allgemeinen Verbindungen der Formel I bevorzugt, welche in R¹ und R² zusammen mindestens 7, insbesondere mindestens 10 Kohlenstoffatome aufweisen.

Die Verbindungen der Formel I können erfindungsgemäss dadurch hergestellt werden, dass man
a) eine Verbindung der allgemeinen Formel 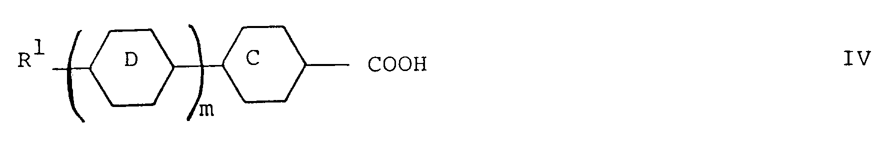 und eine Verbindung der allgemeinen Formel 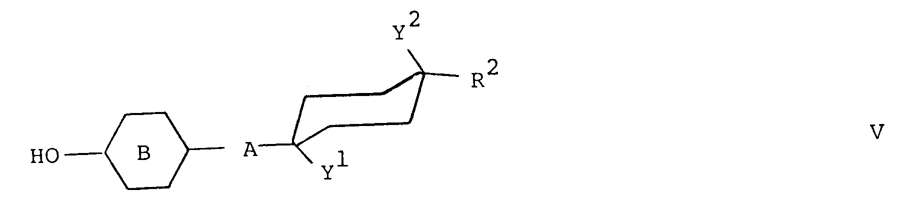 worin A, R¹, R², Y¹, Y², m und die Ringe B, C und D die oben angegebenen Bedeutungen haben, oder reaktionsfähige Derivate dieser Verbindungen verestert, und dass man gewünschtenfalls eine erhaltene Verbindung der Formel I, worin Ring B, C oder D mit Chlor oder Brom substituiertes 1,4-Phenylen bedeutet, mit Kupfer-(I)-cyanid, Natriumcyanid oder Kaliumcyanid umsetzt, oder
b) zur Herstellung der Verbindungen der Formel I, worin A eine Estergruppe -COO- oder -OOC- bedeutet, eine Verbindung der allgemeinen Formel 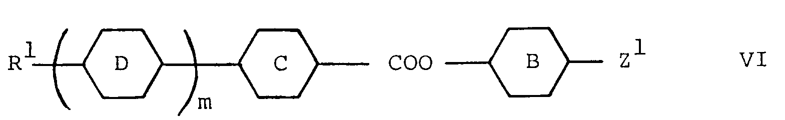 und eine Verbindung der allgemeinen Formel 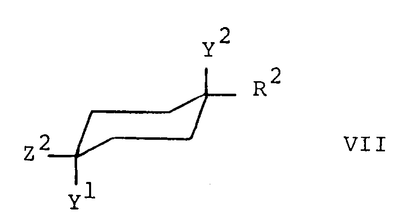 worin eine der Gruppen Z¹ und Z² die Carboxylgruppe und die andere die Hydroxygruppe bezeichnet, und R¹, R², Y¹, Y², m und die Ringe B, C und D die oben angegebenen Bedeutungen haben,
   oder reaktionsfähige Derivate dieser Verbindungen verestert, und dass man gewünschtenfalls eine erhaltene Verbindung der Formel I, worin Ring B, C oder D mit Chlor oder Brom substituiertes 1,4-Phenylen bedeutet, mit Kupfer-(I)-cyanid, Natriumcyanid oder Kaliumcyanid umsetzt.

Die Umsetzung der Verbindungen der Formeln IV und V bzw. der Verbindungen der Formeln VI und VII kann in an sich bekannter Weise durch Veresterung der Carbonsäure oder eines reaktionsfähigen Derivates (z.B. Säurechlorid, -bromid oder -anhydrid) mit der Hydroxyverbindung oder eines geeigneten Salzes (z.B. des Natriumsalzes) durchgeführt werden. Eine bevorzugte Methode ist die Umsetzung des Säurechlorids (welches aus der Carbonsäure z.B. durch Erhitzen mit Thionylchlorid erhältlich ist) mit der Hydroxyverbindung. Diese Umsetzung wird zweckmässig in einem inerten organischen Lösungsmittel, beispielsweise Diäthyläther, Tetrahydrofuran, Dimethylformamid, Benzol, Toluol, Cyclohexan, Tetrachlorkohlenstoff und dergleichen, durchgeführt. Um den bei der Reaktion frei werdenden Chlorwasserstoff zu binden, benützt man zweckmässig ein Säurebindemittel, beispielsweise ein tertiäres Amin, Pyridin und dergleichen. Das Säurebindemittel kann auch zugleich als Lösungsmittel dienen. Weitere bevorzugte Methoden sind die Umsetzung der Carbonsäure mit der Hydroxyverbindung in Gegenwart von 4-(Dimethylamino)pyridin und N,N'-Dicyclohexylcarbodiimid oder in Gegenwart von Oxalylchlorid und Dimethylformamid. Temperatur und Druck der obigen Veresterungsreaktionen sind nicht kritisch und im allgemeinen werden Atmosphärendruck und eine Temperatur zwischen -30°C und der Siedetemperatur des Reaktionsgemisches angewendet.

Die Umsetzung einer Verbindung der Formel I, worin Ring B, C oder D mit Chlor oder Brom substituiertes 1,4-Phenylen bedeutet, zur entsprechenden cyanosubstituierten Verbindung wird zweckmässig mit Kupfer-(I)-cyanid, Natriumcyanid oder Kaliumcyanid in einem inerten organischen Lösungsmittel, wie Aethylenglykol, Tetrahydrofuran, Dimethylformamid, Dimethylsulfoxid, Pyridin oder Acetonitril, durchgeführt. Temperatur und Druck sind nicht kritisch. Zweckmässigerweise werden Atmosphärendruck und eine Temperatur zwischen Raumtemperatur und der Siedetemperatur des Reaktionsgemisches angewendet.

Die Ausgangsmaterialien der Formeln IV-VII sind bekannte oder Analoge bekannter Verbindungen und können nach bekannten Methoden hergestellt werden.

Die erfindungsgemässen Verbindungen können in Form von Gemischen untereinander und/oder mit anderen Flüssigkristallkomponenten verwendet werden. Sie eignen sich insbesondere zur Herstellung von chiral smektischen Gemischen. vor allem für Gemische mit einer smektisch C Phase. Verbindungen mit ferroelektrischen Eigenschaften, die sich als Mischungskomponenten eignen können, sind beispielsweise die in Patent Abstracts of Japan C Sektion, Band 9, Nr. 7, Seite 148 C 262 Nr. 59-167 542 offenbarten, optisch aktiven 3- und 4-kernigen Cyclohexylen Derivate, welche am Cyclohexylenring eine axiale Methylgruppe tragen oder auch die in Patent Abstracts of Japan C Sektion, Band 7, Nr. 252, Seite 146 C 194 Nr. 58-140 045 offenbarten, optisch inaktiven 4-kernigen Verbindungen. Die erfindungsgemässen Verbindungen können aber grundsätzlich auch als Zusätze für nematische Gemische (im Falle optisch inaktiver Verbindungen) oder cholesterische Gemische verwendet werden. Die erfindungsgemässen Gemische enthalten mindestens 2 Kompoenten, wovon mindestens eine Komponente eine Verbindung der Formel I ist. Der Anteil der Verbindungen der Formel I in den erfindungsgemässen Gemischen kann je nach Verwendungszweck in breiten Grenzen variieren und beispielsweise etwa 1% bis 100% betragen. Im allgemeinen enthalten die erfindungsgemässen Mischungen mit nematischer cholesterischer oder chiral smektischer Phase etwa 1-80 Gew.-%, vorzugsweise etwa 5-50 Gew.-% an Verbindungen der Formel I. Ein besonders bevorzugter Bereich für chiral smektische Gemische beträgt etwa 10-70 Gew.-%, insbesondere etwa 30-50 Gew.-%. Bei Verwendung optisch aktiver Verbindungen der Formel I als Zusätze zu Gemischen für Drehzellenanwendungen kann ihr Anteil auch geringer sein und in Abhängigkeit der Ganghöhe und der Zellendicke beispielsweise etwa 0,2-10 Gew.-% betragen.

Die erfindungsgemässen Flüssigkristallmischungen mit nematischen oder cholesterischen Eigenschaften und die Gemische für Drehzellenanzeigen enthalten neben einer oder mehreren Verbindungen der Formel I vorzugsweise eine oder mehrere der Verbindungen der folgenden allgemeinen Formeln 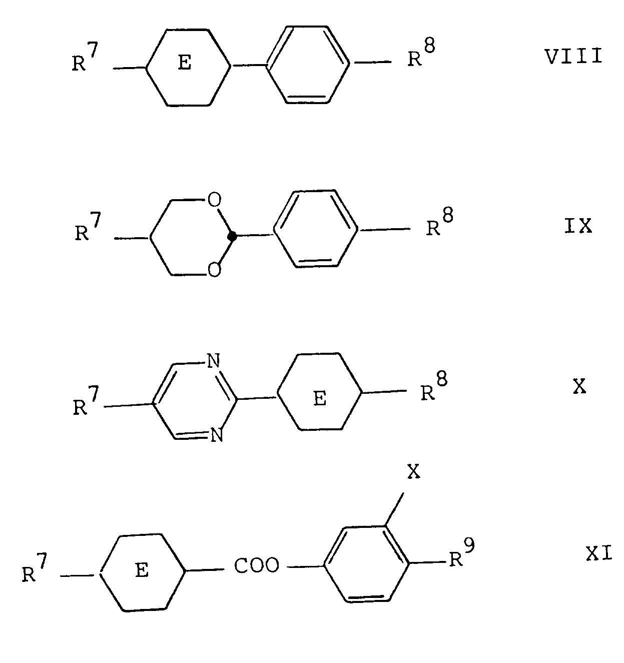 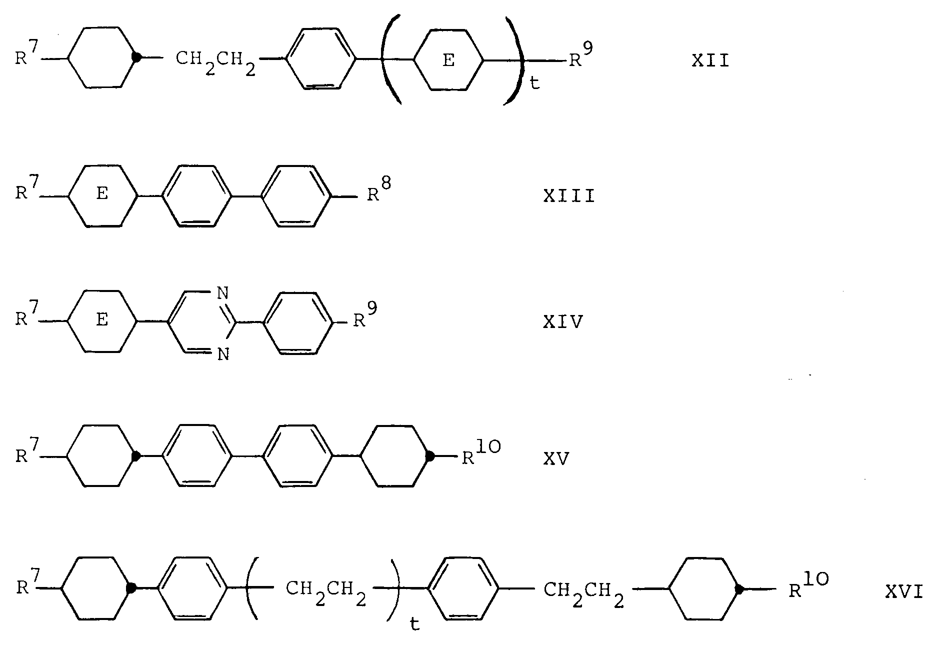
worin R⁷, R⁸, R⁹ und R¹⁰ unabhängig voneinander Alkyl, Alkoxy, Alkenyl oder Alkenyloxy mit höchstens je 7 Kohlenstoffatomen bedeuten oder R⁸ an einem Benzolring auch Cyano oder -NCS bedeutet oder R⁹ an einem Benzolring auch Cyano bedeutet; Ring E trans-1,4-Cyclohexylen oder 1,4-Phenylen darstellt; X Wasserstoff oder Fluor bezeichnet; und t für die Zahl 0 oder 1 steht.

Die erfindungsgemässen Flüssigkristallmischungen mit chiral smektischen Phasen können neben einer oder mehreren Verbindungen der Formel I übliche Komponenten für chiral smektische Gemische enthalten. Vorzugsweise enthalten sie eine oder mehrere Verbindungen der allgemeinen Formeln 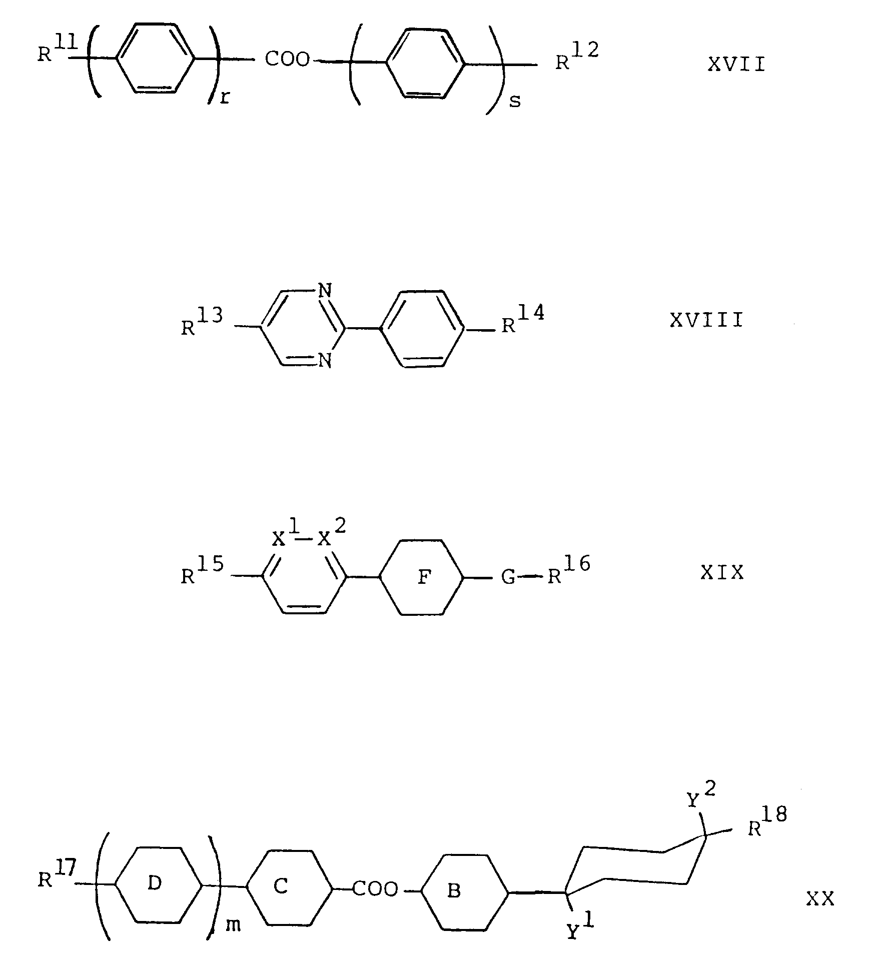
worin R¹¹ und R¹² Alkyl, Alkoxy, Alkanoyl, Alkanoyloxy, Alkoxycarbonyl oder Alkoxycarbonyloxy mit bis zu 18 Kohlenstoffatomen bedeuten; r und s die Zahlen 1 oder 2 bezeichnen; R¹³ und R¹⁴ Alkyl oder Alkoxy mit 1-18 Kohlenstoffatomen darstellen; X¹ für CH und X² für N steht oder X¹ für N und X² für CH steht; G eine einfache Kovalenzbindung, trans-1,4-Cyclohexylen, cis--4-Cyano-trans-1,4-cyclohexylen oder gegebenenfalls mit Halogen oder Methyl substituiertes 1,4-Phenylen bedeutet; Ring F trans-1,4-Cyclohexylen, gegebenenfalls mit Halogen oder Methyl substituiertes 1,4-Phenylen oder, wenn G eine einfache Kovalenzbindung bedeutet, auch cis-4-Cyano-trans-1,4-cyclohexylen darstellt; R¹⁵ und R¹⁶ je eine gegebenenfalls halogensubstituierte Alkyl- oder Alkenylgruppe bezeichnen, in welcher gegebenenfalls eine oder zwei nicht benachbarte CH₂-Gruppen durch -O-, -COO- und/oder -OOC- ersetzt sind; m, Y¹, Y² und die Ringe B, C und D die in Formel I gegebenen Bedeutungen haben; und R¹⁷ und R¹⁸ C₁-C₁₈-Alkyl darstellen, in welchem gegebenenfalls eine oder zwei nicht benachbarte CH₂-Gruppen durch Sauerstoff ersetzt sind.

Die erfindungsgemässen, chiral smektischen Gemische können grundsätzlich aus optisch inaktiven Verbindungen bestehen. Vorzugsweise enthalten sie jedoch eine oder mehrere optisch aktive Verbindungen zwecks Erzielung einer spontanen Polarisation, d.h. sie enthalten vorzugsweise mindestens eine optisch aktive Verbindung der Formel I mit chiralem Kohlenstoffatom in R¹ und/oder R² und/oder mindestens einen optisch aktiven Zusatz. Bevorzugte, chiral smektische Gemische mit mindestens 2 Komponenten sind somit diejenigen, worin mindestens eine Komponente eine optisch aktive Verbindung der Formel I ist, und eine zweite Komponente optisch aktiv oder optisch inaktiv sein kann, sowie diejenigen, worin mindestens eine Komponente eine optisch inaktive, vorzugsweise achirale Verbindung der Formel I ist und eine zweite Komponente optisch aktiv ist. Die zweite Komponente ist vorzugsweise eine weitere Verbindung der Formel I oder eine Verbindung der Formeln XVII-XX.

Die optisch aktiven Verbindungen der Formel XX und die an B, C und/oder D lateral substituierten und/oder am Cyclohexanring axial substituierten Verbindungen der Formel XX sind neu. Sie können in analoger Weise zu den Verbindungen der Formel I hergestellt werden.

Die erfindungsgemässen Mischungen können ebenfalls dichroitische Farbstoffe enthalten, beispielsweise Azo-, Azoxy- oder Anthrachinonfarbstoffe. Der Anteil der Farbstoffe wird durch die Löslichkeit und die gewünschte Farbe, Extinktion und dergleichen bestimmt und beträgt im allgemeinen höchstens etwa 10 Gew.-% im Gesamtgemisch.

Die Herstellung der erfindungsgemässen Mischungen und der elektro-optischen Vorrichtungen kann in an sich bekannter Weise erfolgen.

Die Herstellung der erfindungsgemässen Verbindungen, der neuen Verbindungen der Formel XX und der Ausgangsmaterialien sowie erfindungsgemässe Mischungen werden durch die folgenden Beispiele weiter veranschaulicht. Die Phasen werden durch die folgenden Symbole bezeichnet: C für kristallin, S für smektisch, S_{A} für smektisch A, S_{B} für smektisch B, S_{C} für smektisch C, S*_{C} für chiral smektisch C, Ch für cholesterisch, N für nematisch und I für isotrop.

### Beispiel 1

Eine Lösung von 2,6 g Bortribromid in 50 ml absolutem Dichlormethan wurde bei 0°C tropfenweise mit einer Lösung von 2,0 g 2-(trans-4-Pentylcyclohexyl)-1-(4-methoxyphenyl)äthan in 50 ml absolutem Dichlormethan versetzt. Das Gemisch wurde 1 Stunde gerührt und dann auf Eiswasser gegossen. Die organische Phase wurde abgetrennt und die wässrige Phase dreimal mit je 50 ml Dichlormethan nachextrahiert. Die vereinigten organischen Phasen wurden mit 50 ml 2N Natriumcarbonat-Lösung und mehrmals mit Wasser gewaschen, über Magnesiumsulfat getrocknet und eingeengt. Umkristallisation des erhaltenen Rohprodukts aus Hexan bei 0°C ergab 1,8 g 4-[2-(trans-4-Pentylcyclohexyl)äthyl]phenol mit Smp. 102°C.

### Beispiel 2

Ein Gemisch von 5,0 g (trans-4-Heptylcyclohexyl)methylbromid, 10,0 g Hydrochinon, 10,0 g wasserfreiem Kaliumcarbonat und 100 ml absolutem Dimethylformamid wurde über Nacht unter Rückfluss erhitzt. Anschliessend wurde das abgekühlte Reaktionsgemisch auf Eiswasser gegossen und dreimal mit je 50 ml Diäthyläther extrahiert. Die organischen Phasen wurden mit 500 ml Wasser gewaschen, über Magnesiumsulfat getrocknet und eingeengt. Chromatographie des Rückstandes an Kieselgel mit Toluol/Aethylacetat (Vol. 4:1) ergab 1,8 g 4-[(trans-4-Heptylcyclohexyl)methoxy]phenol; Smp. 107-108°C.

### Beispiel 3

Aus 2,2 g 2-(trans-4-Pentylcyclohexyl)-1-(2,3-dicyano-4-butyloxyphenyl)äthan, 1,4 g wasserfreiem Aluminiumchlorid und 0,3 g Natriumchlorid wurde eine feinpulverisierte Mischung bereitet und diese 40 Minuten unter wasserfreien Bedingungen auf dem Oelbad auf 150°C erhitzt. Die abgekühlte Mischung wurde mit 250 ml Wasser versetzt und dreimal mit je 50 ml Dichlormethan extrahiert. Die vereinigten organischen Phasen wurden mit 250 ml Wasser gewaschen, über Magnesiumsulfat getrocknet und eingeengt. Chromatographie des Rückstandes an Kieselgel mit Toluol/Aethylacetat (Vol. 4:1) ergab 0,6 g 2,3-Dicyano-4-[2-(trans-4-pentylcyclohexyl)äthyl)phenol; Smp. 100-102°C.

### Beispiel 4

Eine Lösung von 2,7 g 4-[2-(trans-4-Pentylcyclohexyl)-äthyl]phenol in absolutem Dichlormethan wurde bei 0°C tropfenweise mit einer Lösung von 1,6 g Brom in 20 ml absolutem Dichlormethan versetzt. Das Gemisch wurde 1 Stunde gerührt und dann auf Eiswasser gegossen. Die organische Phase wurde abgetrennt und die wässrige Phase dreimal mit je 50 ml Dichlormethan nachextrahiert. Die vereinigten organischen Phasen wurden mit 50 ml 2N Natriumcarbonat-Lösung und mehrmals mit Wasser gewaschen, über Magnesiumsulfat getrocknet und eingeengt. Umkristallisation des erhaltenen Rohprodukts aus Hexan bei 0°C ergab 3,4 g 2-Brom-4-[2-(trans-4--pentylcyclohexyl)äthyl]phenol.

### Beispiel 5

Ein Gemisch von 0,25 g (S)-4-(6-Methyloctyloxy)benzoesäure, 5 ml Thionylchlorid (Ueberschuss) und 20 ml Toluol wurde 1 Stunde zum Sieden erhitzt. Anschliessend wurde das Lösungsmittel und überschüssiges Thionylchlorid abdestilliert und der Rückstand zweimal in je 25 ml Toluol aufgenommen und eingeengt.

Das erhaltene, rohe (S)-4-(6-Methyloctyloxy)benzoesäurechlorid wurde in 20 ml Toluol gelöst und dann zu einer Lösung von 0,26 g 4-[2-(trans-4-Pentylcyclohexyl)äthyl]phenol in 2 ml Pyridin zugetropft. Das Reaktionsgemisch wurde über Nacht bei Raumtemperatur gerührt, dann auf Eiswasser gegossen und mit Diäthyläther extrahiert. Der Extrakt wurde viermal mit je 25 ml 3N Salzsäure gewaschen, dann mit 25 ml 2N Natriumcarbonat-Lösung und mehrmals mit Wasser gewaschen, über Magnesiumsulfat getrocknet und eingeengt. Chromatogrphie des zurückbleibenden Oeles an Kieselgel mit Toluol ergab 0,2 g (S)-4-(6-Methyloctyloxy)benzoesäure-4-[2-(trans -4-pentylcyclohexyl)äthyl]phenylester. Das Produkt wurde mehrmals aus Aethanol umkristallisiert, bis die Phasenübergangstemperaturen konstant waren. Smp. (C-S) 58°, Umwandlung S-S*_{C} 58,5°C, Umwandlung S*_{C}-Ch 93°C, Klp. (Ch-I) 140°C.

In analoger Weise können folgende Verbindungen hergestellt werden:
(S)-4-(2-Methylbutyloxy)benzoesäure-4-[2-(trans-4-propylcyclohexyl)äthyl]phenylester, Smp. (C-Ch) 81°C, Klp. (Ch-I) 146°C;
(S)-4-(3-Methylpentyloxy)benzoesäure-4-[2-(trans-4-propylcyclohexyl)äthyl]phenylester,
(S)-4-(4-Methylhexyloxy)benzoesäure-4-[2-(trans-4-propylcyclohexyl)äthyl]phenylester, Smp. (C-Ch) 82°C, Klp. (Ch-I) 149°C;
(S)-4-(5-Methylheptyloxy)benzoesäure-4-[2-(trans-4-propylcyclohexyl)äthyl]phenylester,
(S)-4-(6-Methyloctyloxy)benzoesäure-4-[2-(trans-4-propylcyclohexyl)äthyl]phenylester, Smp. (C-S*_{C}) 73°C, Umwandlung S-S*_{C} 60,5°C (monotrop), Umwandlung S*_{C}-Ch 77°C, Klp. (Ch-I) 144°C;
(S)-4-(2-Methylbutyloxy)benzoesäure-4-[2-(trans-4-pentylcyclohexyl)äthyl]phenylester, Smp. (C-Ch) 74°C, Klp. (Ch-I) 130°C;
(S)-4-(3-Methylpentyloxy)benzoesäure-4-[2-(trans-4-pentylcyclohexyl)äthyl]phenylester,
(S)-4-(4-Methylhexyloxy)benzoesäure-4-[2-(trans-4-pentylcyclohexyl)äthyl]phenylester, Smp. (C-Ch) 66°C, Klp. (Ch-I) 137°C;
(S)-4-(5-Methylheptyloxy)benzoesäure-4-[2-(trans-4-pentylcyclohexyl)äthyl]phenylester,
(S)-4-(2-Methylbutyloxy)benzoesäure-4-[2-(trans-4-heptylcyclohexyl)äthyl]phenylester, Smp. (C-Ch) 66°C, Klp. (Ch-I) 131°C;
(S)-4-(4-Methylhexyloxy)benzoesäure-4-[2-(trans-4-heptylcyclohexyl)äthyl]phenylester, Smp. (C-S) -6°C, Umwandlung S-S 61,5°C, Umwandlung S-S*_{C} 69,5°C, Umwandlung S*_{C}-Ch 75°C, Klp. (Ch-I) 135,5°C;
(S)-4-(6-Methyloctyloxy)benzoesäure-4-[2-(trans-4-heptylcyclohexyl)äthyl]phenylester, Smp. (C-S) 42°C, Umwandlung S-S 44°C, Umwandlung S-S*_{C} 68,5°C, Umwandlung S*_{C}-Ch 102,5°C, Klp. (Ch-I) 135,5°C;
(S)-4'-(2-Methylbutyl)-4-biphenylcarbonsäure-4-[2-(trans-4-pentylcyclohexyl)äthyl]phenylester, Smp. (C-S_{B}) 96°C, Umwandlung S_{B}-S*_{C} 102°C, Umwandlung S*_{C}-Ch 104°C, Klp. (Ch-I) 238,5°C;
(S)-4'-(4-Methylhexyl)-4-biphenylcarbonsäure-4-[2-(trans-4-pentylcyclohexyl)äthyl]phenylester,
(S)-4'-(2-Methylbutyloxy)-4-biphenylcarbonsäure-4-[2-(trans-4-pentylcyclohexyl)äthyl]phenylester, Smp. (C-S*_{C}) 128°C, Umwandlung S*_{C}-Ch 130°C, Klp. (Ch-I) 247°C;
(S)-4'-(4-Methylhexyloxy)-4-biphenylcarbonsäure-4-[2-(trans-4-pentylcyclohexyl)äthyl]phenylester,
(S)-4-(2-Methylbutyloxy)benzoesäure-4-[(trans-4-propylcyclohexyl)methoxy]phenylester, Smp. (C-Ch) 91,5°C, Klp. (Ch-I) 143,5°C;
(S)-4-(4-Methylhexyloxy)benzoesäure-4-[(trans-4-propylcyclohexyl)methoxy]phenylester, Smp. (C-Ch) 74°C, Klp. (Ch-I) 148°C;
(S)-4-(6-Methyloctyloxy)benzoesäure-4-[(trans-4-propylcyclohexyl)methoxy]phenylester, Smp. (C-S*_{C}) 83°C, Umwandlung S-S*_{C} 68°C, Umwandlung S*_{C}-Ch 94°C, Klp. (Ch-I) 144,5°C;
(S)-4-(2-Methylbutyloxy)benzoesäure-4-[(trans-4-pentylcyclohexyl)methoxy]phenylester, Smp. (C-Ch) 87,5°C, Klp. (Ch-I) 140,5°C;
(S)-4-(4-Methylhexyloxy)benzoesäure-4-[(trans-4-pentylcyclohexyl)methoxy]phenylester, Smp. (C-Ch) 85,5°C, Umwandlung S-S*_{C} 44°C, Umwandlung S*_{C}-Ch 85°C, Klp. (Ch-I) 147,5°C;
(S)-4-(6-Methyloctyloxy)benzoesäure-4-[(trans-4-pentylcyclohexyl)methoxy]phenylester, Smp. (C-S*_{C}) 72,5°C, Umwandlung S-S*_{C} 65°C, Umwandlung S*_{C}-Ch 107,5°C, Klp. (Ch-I) 145,5°C;
(S)-4-(2-Methylbutyloxy)benzoesäure-4-[(trans-4-heptylcyclohexyl)methoxy]phenylester, Smp. (C-Ch) 66,5°C, Umwandlung S-S*_{C} 63°C, Umwandlung S*_{C}-Ch 65°C, Klp. (Ch-I) 135,5°C;
(S)-4-(3-Methylpentyloxy)benzoesäure-4-[(trans-4-heptylcyclohexyl)methoxy]phenylester,
(S)-4-(4-Methylhexyloxy)benzoesäure-4-[(trans-4-heptylcyclohexyl)methoxy]phenylester, Smp. (C-S*_{C}) 77°C, Umwandlung S*_{C}-Ch 92,5°C, Klp. (Ch-I) 142°C;
(S)-4-(5-Methylheptyloxy)benzoesäure-4-[(trans-4-heptylcyclohexyl)methoxy]phenylester,
(S)-4-(6-Methyloctyloxy)benzoesäure-4-[(trans-4-heptylcyclohexyl)methoxy]phenylester, Smp. (C-S) 72°C, Umwandlung S-S*_{C} 74°C, Umwandlung S*_{C}-CH 114,5°C, Klp. (Ch-I) 142°C;
(S)-4-(4-Methylhexyloxy)benzoesäure-2,3-dicyano-4-[2-(trans-4-pentylcyclohexyl)äthyl]phenylester,
(S)-4-(6-Methyloctyloxy)benzoesäure-2,3-dicyano-4-[2-(trans-4-pentylcyclohexyl)äthyl]phenylester, Smp. (C-I) 123°C, Umwandlung S*_{C}-Ch 113°C, Klp. (Ch-I) 116°C;
(S)-4-(6-Methyloctyloxy)benzoesäure-2-brom-4-[2-(trans-4-pentylcyclohexyl)äthyl]phenylester, Smp. (C-Ch) 57°C, Klp. (Ch-I) 97°C;
(S)-4-(2-Methylbutoxy)benzoesäure-4-[2-(trans-4-[2-propenyl]cyclohexyl)äthyl]phenylester,
(S)-4-(4-Methylhexyloxy)benzoesäure-4-[2-(trans-4-[2-propenyl]cyclohexyl)äthyl]phenylester,
(S)-4-(6-Methyloctyloxy)benzoesäure-4-[2-(trans-4-[2-propenyl]cyclohexyl)äthyl]phenylester,
(S)-4-(6-Methyloctyloxy)benzoesäure-4-[2-(trans-4-[1E-pentenyl]cyclohexyl)äthyl]phenylester,
(S)-4-(6-Methyloctyloxy)benzoesäure-4-[2-(trans-4-[2Z-pentenyl]cyclohexyl)äthyl]phenylester,
(S)-4-(6-Methyloctyloxy)benzoesäure-4-[2-(trans-4-[3E-pentenyl]cyclohexyl)äthyl]phenylester,
(S)-4-(6-Methyloctyloxy)benzoesäure-4-[2-(trans-4-[4-pentenyl]cyclohexyl)äthyl]phenylester,
(S)-4-(6-Methyloctyloxy)benzoesäure-4-[2-(trans-4-[1E-heptenyl]cyclohexyl)äthyl]phenylester,
(S)-4-(6-Methyloctyloxy)benzoesäure-4-[2-(trans-4-[2Z-heptenyl]cyclohexyl)äthyl]phenylester,
(S)-4-(6-Methyloctyloxy)benzoesäure-4-[2-(trans-4-[3E-heptenyl]cyclohexyl)äthyl]phenylester,
(S)-4-(6-Methyloctyloxy)benzoesäure-4-[2-(trans-4-[4Z-heptenyl]cyclohexyl)äthyl]phenylester,
(S)-4-(6-Methyloctyloxy)benzoesäure-4-[2-(trans-4-[5E-heptenyl]cyclohexyl)äthyl]phenylester,
(S)-4-(6-Methyloctyloxy)benzoesäure-4-[2-(trans-4-[6-heptenyl]cyclohexyl)äthyl]phenylester,
(S)-4-(6-Methyloctyloxy)benzoesäure-4-[(trans-4-[1E-pentenyl]cyclohexyl)methoxy]phenylester,
(S)-4-(6-Methyloctyloxy)benzoesäure-4-[(trans-4-[2Z-pentenyl]cyclohexyl)methoxy]phenylester,
(S)-4-(6-Methyloctyloxy)benzoesäure-4-[(trans-4-[3E-pentenyl]cyclohexyl)methoxy]phenylester,
(S)-4-(6-Methyloctyloxy)benzoesäure-4-[(trans-4-[4-pentenyl]cyclohexyl)methoxy]phenylester,
(S)-4-(6-Methyloctyloxy)benzoesäure-4-(trans-4-[1E-pentenyl]cyclohexyl)phenylester,
(S)-4-(6-Methyloctyloxy)benzoesäure-4-(trans-4-[2Z-pentenyl]cyclohexyl)phenylester,
(S)-4-(6-Methyloctyloxy)benzoesäure-4-(trans-4-[3E-pentenyl]cyclohexyl)phenylester,
(S)-4-(6-Methyloctyloxy)benzoesäure-4-(trans-4-[4-pentenyl]cyclohexyl)phenylester,
4-Hexyloxybenzoesäure-4-[2-(trans-4-propylcyclohexyl)äthyl]phenylester,
4-Heptyloxybenzoesäure-4-[2-(trans-4-propylcyclohexyl)äthyl]phenylester,
4-Octyloxybenzoesäure-4-[2-(trans-4-propylcyclohexyl)äthyl]phenylester,
3-Nonyloxybenzoesäure-4-[2-(trans-4-propylcyclohexyl)äthyl]phenylester,
4-Decyloxybenzoesäure-4-[2-(trans-4-propylcyclohexyl)äthyl]phenylester,
4-Undecyloxybenzoesäure-4-[2-(trans-4-propylcyclohexyl)äthyl]phenylester,
4-Dodecyloxybenzoesäure-4-[2-(trans-4-propylcyclohexyl)äthyl]phenylester,
4-Hexyloxybenzoesäure-4-[2-(trans-4-pentylcyclohexyl)äthyl]phenylester,
4-Heptyloxybenzoesäure-4-[2-(trans-4-pentylcyclohexyl)äthyl]phenylester, Smp. (C-S_{C}) 67°C, Umwandlung S-S_{C} 51°C, Umwandlung S_{C}-N 67,5°C, Klp. (N-I) 160°C;
4-Octyloxybenzoesäure-4-[2-(trans-4-pentycyclohexyl)äthyl]phenylester, Smp. (C-S_{C}) 68°C, Umwandlung S-S_{C} 57°C, Umwandlung S_{C}-N 86,5°C, Klp. (N-I) 157,5°C;
4-Nonyloxybenzoesäure-4-[2-(trans-4-pentylcyclohexyl)äthyl]phenylester, Smp. (C-S_{C}) 61°C, Umwandlung S-S_{C} 57°C, Umwandlung S_{C}-N 101°C, Klp. (N-I) 153,5°C;
4-Decyloxybenzoesäure-4-[2-(trans-4-pentylcyclohexyl)äthyl]phenylester, Smp. (C-S_{C}) 60°C, Umwandlung S-S_{C} 57,5°C, Umwandlung S_{C}-N 106,5°C, Klp. (N-I) 152,5°C;
4-Undecyloxybenzoesäure-4-[2-(trans-4-pentylcyclohexyl)äthyl]phenylester,
4-Dodecyloxybenzoesäure-4-[2-(trans-4-pentylcyclohexyl)äthyl]phenylester, Smp. (C-S) 58°C, Umwandlung S-S_{C} 78°C, Umwandlung S_{C}-S_{A} 118°C, Umwandlung S_{A}-N 131,5°C, Klp. (N-I) 147°C:
4-Heptyloxybenzoesäure-4-[2-(trans-4-heptylcyclohexyl)äthyl]phenylester,
4-Octyloxybenzoesäure-4-[2-(trans-4-heptylcyclohexyl)äthyl]phenylester,
4-Nonyloxybenzoesäure-4-[2-(trans-4-heptylcyclohexyl)äthyl]phenylester,
4-Decyloxybenzoesäure-4-[2-(trans-4-heptylcyclohexyl)äthyl]phenylester,
4-Undecyloxybenzoesäure-4-[2-(trans-4-heptylcyclohexyl)äthyl]phenylester,
4-Dodecyloxybenzoesäure-4-[2-(trans-4-heptylcyclohexyl)äthyl]phenylester,
4-(5-Hexenyloxy)benzoesäure-4-[2-(trans-4-propylcyclohexyl)äthyl]phenylester,
4-(6-Heptenyloxy)benzoesäure-4-[2-(trans-4-propylcyclohexyl)äthyl]phenylester,
4-(7-Octenyloxy)benzoesäure-4-[2-(trans-4-propylcyclohexyl)äthyl]phenylester,
4-(8-Nonenyloxy)benzoesäure-4-[2-(trans-4-propylcyclohexyl)äthyl]phenylester,
4-(9-Decenyloxy)benzoesäure-4-[2-(trans-4-propylcyclohexyl)äthyl]phenylester,
4-(10-Undecenyloxy)benzoesäure-4-[2-(trans-4-propylcyclohexyl)äthyl]phenylester,
4-(11-Dodecenyloxy)benzoesäure-4-[2-(trans-4-propylcyclohexyl)äthyl]phenylester,
4-(5-Hexenyloxy)benzoesäure-4-[2-(trans-4-pentylcyclohexyl)äthyl]phenylester,
4-(6-Heptenyloxy)benzoesäure-4-[2(trans-4-pentylcyclohexyl)äthyl]phenylester,
4-(7-Octenyloxy)benzoesäure-4-[2-(trans-4-pentylcyclohexyl)äthyl]phenylester, Smp. (C-S_{C}) 62°C, Umwandlung S-S_{C} 43,5°C, Umwandlung S_{C}-N 73°C, Klp. (N-I) 152,5°C;
4-(8-Nonenyloxy)benzoesäure-4-[2-(trans-4-pentylcyclohexyl)äthyl]phenylester, Smp. (C-S_{C}) 55°C, Umwandlung S-S_{C} 44°C, Umwandlung S_{C}-N 91,5°C, Klp. (N-I) 153°C;
4-(9-Decenyloxy)benzoesäure-4-[2-(trans-4-pentylcyclohexyl)äthyl]phenylester, Smp. (C-S_{C}) 69°C, Umwandlung S-S_{C} 57°C, Umwandlung S_{C}-S_{A} 104°C, Umwandlung S_{A}-N 110°C, Klp. (N-I) 148°C;
4-(10-Undecenyloxy)benzoesäure-4-[2-(trans-4-pentylcyclohexyl)äthyl]phenylester, Smp. (C-S) 51°C, Umwandlung S-S_{C} 69°C, Umwandlung S_{C}-S_{A} 100,5°C, Umwandlung S_{A}-N 124°C, Klp. (N-I) 147,5°C;
4-(11-Dodecenyloxy)benzoesäure-4-[2-(trans-4-pentylcyclohexyl)äthyl]phenylester,
4-(5-Hexenyloxy)benzoesäure-4-[2-(trans-4-heptylcyclohexyl)äthyl]phenylester,
4-(6-Heptenyloxy)benzoesäure-4-[2-(trans-4-heptylcyclohexyl)äthyl]phenylester,
4-(7-Octenyloxy)benzoesäure-4-[2-(trans-4-heptylcyclohexyl)äthyl]phenylester,
4-(8-Nonenyloxy)benzoesäure-4-[2-(trans-4-heptylcyclohexyl)äthyl]phenylester,
4-(9-Decenyloxy)benzoesäure-4-[2-(trans-4-heptylcyclohexyl)äthyl]phenylester,
4-(10-Undecenyloxy)benzoesäure-4-[2-(trans-4-heptylcyclohexyl)äthyl]phenylester,
4-(11-Dodecenyloxy)benzoesäure-4-[2-(trans-4-heptylcyclohexyl)äthyl]phenylester,
4-Hexyloxybenzoesäure-2-cyano-4-[2-(trans-4-pentylcyclohexyl)äthyl]phenylester,
4-Heptyloxybenzoesäure-2-cyano-4-[2-(trans-4-pentylcyclohexyl)äthyl]phenylester,
4-Octyloxybenzoesäure-2-cyano-4-[2-(trans-4-pentylcyclohexyl)äthyl]phenylester,
4-Nonyloxybenzoesäure-2-cyano-4-[2-(trans-4-pentylcyclohexyl)äthyl]phenylester,
4-Decyloxybenzoesäure-2-cyano-4-[2-(trans-4-pentylcyclohexyl)äthyl]phenylester,
4-Undecyloxybenzoesäure-2-cyano-4-[2-(trans-4-pentylcyclohexyl)äthyl]phenylester,
4-Dodecyloxybenzoesäure-2-cyano-4-[2-(trans-4-pentylcyclohexyl)äthyl]phenylester, Smp. (C-S_{C}) 62°C, Umwandlung S_{C}-N 66°C, Klp. (N-I) 120,5°C;
4-Hexyloxybenzoesäure-2-cyano-4-[2-(trans-4-heptylcyclohexyl)äthyl]phenylester,
4-Heptyloxybenzoesäure-2-cyano-4-[2-(trans-4-heptylcyclohexyl)äthyl]phenylester,
4-Octyloxybenzoesäure-2-cyano-4-[2-(trans-4-heptylcyclohexyl)äthyl]phenylester,
4-Nonyloxybenzoesäure-2-cyano-4-[2-(trans-4-heptylcyclohexyl)äthyl]phenylester,
4-Decyloxybenzoesäure-2-cyano-4-[2-(trans-4-heptylcyclohexyl)äthyl]phenylester,
4-Undecyloxybenzoesäure-2-cyano-4-[2-(trans-4-heptylcyclohexyl)äthyl]phenylester,
4-Dodecyloxybenzoesäure-2-cyano-4-[2-(trans-4-heptylcyclohexyl)äthyl]phenylester,
4-Octyloxybenzoesäure-4-[(trans-4-pentylcyclohexyl)methoxy]phenylester,
4-Octyloxybenzoesäure-4-[(trans-4-(1E-pentenyl)methoxy]phenylester,
4-Octyloxybenzoesäure-4-[(trans-4-(4-pentenyl)methoxy]phenylester,
4-(7-Octenyloxy)benzoesäure-4-[(trans-4-pentylcyclohexyl)methoxy]phenylester,
4-(8-Nonenyloxy)benzoesäure-4-[(trans-4-pentylcyclohexyl)methoxy]phenylester,
4-(9-Decenyloxy)benzoesäure-4-[(trans-4-pentylcyclohexyl)methoxy]phenylester,
(S)-4-(6-Methyloctyloxy)benzoesäure-4-[trans-4-(1E-pentenyl)cyclohexyl]phenylester,
(S)-4-(6-Methyloctyloxy)benzoesäure-4-[trans-4-(4-pentenyl)cyclohexyl]phenylester,
4-Octyloxybenzoesäure-4-[trans-4-(1E-pentenyl)cyclohexyl]phenylester,
4-Octyloxybenzoesäure-4-[trans-4-(4E-pentenyl)cyclohexyl]phenylester,
4-(7-Octenyloxy)benzoesäure-4-[trans-4-pentylcyclohexyl]phenylester,
4-(8-Nonenyloxy)benzoesäure-4-[trans-4-pentylcyclohexyl]phenylester,
4-(9-Decenyloxy)benzoesäure-4-[trans-4-pentylcyclohexyl]phenylester,
4-(5-Hexenyloxy)benzoesäure-2-cyano-4-[2-(trans-4-pentylcyclohexyl)äthyl]phenylester,
4-(6-Heptenyloxy)benzoesäure-2-cyano-4-[2-(trans-4-pentylcyclohexyl)äthyl]phenylester,
4-(7-Octenyloxy)benzoesäure-2-cyano-4-[2-(trans-4-pentylcyclohexyl)äthyl]phenylester,
4-(8-Nonenyloxy)benzoesäure-2-cyano-4-[2-(trans-4-pentylcyclohexyl)äthyl]phenylester,
4-(9-Decenyloxy)benzoesäure-2-cyano-4-[2-(trans-4-pentylcyclohexyl)äthyl]phenylester,
4-(10-Undecenyloxy)benzoesäure-2-cyano-4-[2-(trans-4-pentylcyclohexyl)äthyl]phenylester,
4-(11-Dodecenyloxy)benzoesäure-2-cyano-4-[2-trans-4-pentylcyclohexyl)äthyl]phenylester.

### Beispiel 6

Ein Gemisch von 2 g (S)-4-(6-Methyloctyloxy)benzoesäure--2-brom-4-[2-(trans -4-pentylcyclohexyl)äthyl]phenylester, 0,5 g wasserfreiem Kupfer-(I)-cyanid und 50 ml absolutem 1-Methyl-2-pyrrolidon wurde 2 Stunden auf dem Oelbad auf 185°C erhitzt. Anschliessend wurde das abgekühlte Gemisch mit 50 ml 15%-iger Ammoniak-Lösung versetzt und 30 Minuten gerührt. Danach wurde das Reaktionsgemisch dreimal mit je 50 ml Diäthyläther extrahiert. Die vereinigten organischen Phasen wurden zweimal mit je 250 ml Wasser gewaschen, über Magnesiumsulfat getrocknet und eingeengt. Chromatographie des Rückstandes an Kieselgel mit Toluol/Hexan (Vol. 1:1) ergab 0,1 g (S)-4-(6-Methyloctyloxy)benzoesäure-2-cyano-4--[2-(trans -4-pentylcyclohexyl)äthyl]phenylester; Smp. (C-Ch) 80°C, Umwandlung S*_{C}-Ch 50°C (monotrop), Klp. (Ch-I) 120°C.

### Beispiel 7

2,5 g (S)-4-(6-Methyloctyloxy)benzoesäure wurden mit 25 ml Thionylchlorid in Toluol 1 Stunde auf 80°C erhitzt. Die erhaltene Lösung wurde unter vermindertem Druck eingedampft, der Rückstand mit 20 ml absolutem Toluol versetzt und die Lösung nochmals unter vermindertem Druck eingedampft. Das erhaltene Säurechlorid wurde in 50 ml absolutem Toluol aufgenommen und mit einer Lösung von 1,1 g Hyrochinon in 10 ml absolutem Pyridin versetzt. Das Reaktionsgemisch wurde 1 Stunde zum Rückfluss erhitzt, dann abgekühlt, mit verdünnter Salzsäure versetzt und dreimal mit je 50 ml Dichlormethan extrahiert. Die vereinigten organischen Phasen wurden zweimal mit je 250 ml Wasser gewaschen und über Magnesiumsulfat getrocknet und eingeengt. Das erhaltene Rohprodukt wurde durch Säulenchromatographie an Kieselgel mit Toluol/Aethylacetat (Vol. 4:1) gereinigt. Umkristallisation des erhaltenen Produktes aus Aethanol ergab 2.0 g (S)-4-(6-Methyloctyloxy)benzoesäure-4-hydroxyphenylester mit Smp. 75°C.

### Beispiel 8

2,0 g (S)-4-(6-Methyloctyloxy)benzoesäure-4-hydroxyphenylester, 1,1 g trans-4-Pentylcyclohexancarbonsäure und 0,1 g 4-(Dimethylamino)pyridin wurden in 50 ml Dichlormethan gelöst und die Lösung innert 10 Minuten unter Rühren portionenweise mit 1,4 g N,N'-Dicyclohexylcarbodiimid versetzt. Das Gemisch wurde über Nacht bei Raumtemperatur gerührt und dann filtriert. Das Filtrat wurde mit Dichlormethan verdünnt, zweimal mit je 50 ml gesättigter Natriumbicarbonat--Lösung und dann mit Wasser gewaschen, über Natriumsulfat getrocknet und eingeengt. Das erhaltene Rohprodukt wurde an Kieselgel mit Toluol chromatographisch gereinigt. Der erhaltene (S)-4-(6-Methyloctyloxy)benzoesäure-4-(trans -4-pentylcyclohexylcarbonyloxy)phenylester wurde aus Methanol und Aethanol umkristallisiert; Smp. (C-S) 66°C, Umwandlung S-S*_{C} 80°C, Umwandlung S*_{C}-Ch 112°C, Klp. (Ch-I) 186°C.

In analoger Weise können folgende Verbindungen hergestellt werden:
(S)-4-(2-Methylbutyloxy)benzoesäure-4-(trans-4-pentylcyclohexylcarbonyloxy)phenylester,
(S)-4-(4-Methylhexyloxy)benzoesäure-4-(trans-4-pentylcyclohexylcarbonyloxy)phenylester,
(S)-4-(2-Methylbutyl)benzoesäure-4-(trans-4-pentylcyclohexylcarbonyloxy)phenylester,
(S)-4-(4-Methylhexyl)benzoesäure-4-(trans-4-pentylcyclohexylcarbonyloxy)phenylester,
(S)-4-(6-Methyloctyl)benzoesäure-4-(trans-4-pentylcyclohexylcarbonyloxy)phenylester,
(S)-4-(6-Methyloctyloxy)benzoesäure-4-[trans-4-(1E-pentenyl)cyclohexylcarbonyloxy]phenylester,
(S)-4-(6-Methyloctyloxy)benzoesäure-4-[trans-4-(4-pentenyl)cyclohexylcarbonyloxy]phenylester,
(S)-4-(6-Methyloctyloxy)benzoesäure-4-[trans-4-(5-hexenyl)cyclohexylcarbonyloxy]phenylester,
(S)-4-(6-Methyloctyloxy)benzoesäure-4-[trans-4-(6-heptenyl)cyclohexylcarbonyloxy]phenylester,
4-(5-Hexenyloxy)benzoesäure-4-(trans-4-pentylcyclohexylcarbonyloxy)phenylester,
4-(6-Heptenyloxy)benzoesäure-4-(trans-4-pentylcyclohexylcarbonyloxy)phenylester,
4-(7-Octenyloxy)benzoesäure-4-(trans-4-pentylcyclohexylcarbonyloxy)phenylester,
4-(8-Nonenyloxy)benzoesäure-4-(trans-4-pentylcyclohexylcarbonyloxy)phenylester,
4-(9-Decenyloxy)benzoesäure-4-(trans-4-pentylcyclohexylcarbonyloxy)phenylester,
4-Octyloxybenzoesäure-4-[trans-4-(1E-pentenyl)cyclohexylcarbonyloxy]phenylester,
4-Octyloxybenzoesäure-4-[trans-4-(4-pentenyl)cyclohexylcarbonyloxy]phenylester,
4-Octyloxybenzoesäure-4-[trans-4-(5-hexenyl)cyclohexylcarbonyloxy]phenylester,
4-Octyloxybenzoesäure-4-[trans-4-(6-heptenyl)cyclohexylcarbonyloxy]phenylester,
(S)-4-(4-Methylhexyloxy)benzoesäure-4-(trans-4-pentylcyclohexyloxycarbonyl)phenylester,
(S)-4-(4-Methyloctyloxy)benzoesäure-4-(trans-4-pentylcyclohexyloxycarbonyl)phenylester,
(S)-4-(6-Methyloctyloxy)benzoesäure-4-[trans-4-(1E-pentenyl)cyclohexyloxycarbonyl]phenylester,
(S)-4-(6-Methyloctyloxy)benzoesäure-4-[trans-4-(4-pentenyl)cyclohexyloxycarbonyl]phenylester,
(S)-4-(6-Methyloctyloxy)benzoesäure-4-[trans-4-(5-hexenyl)cyclohexyloxycarbonyl]phenylester,
4-(7-Octenyloxy)benzoesäure-4-(trans-4-pentylcyclohexyloxycarbonyl]phenylester,
4-(8-Nonenyloxy)benzoesäure-4-(trans-4-pentylcyclohexyloxycarbonyl]phenylester,
4-(9-Decenyloxy)benzoesäure-4-(trans-4-pentylcyclohexyloxycarbonyl]phenylester,
4-Octyloxybenzoesäure-4-[trans-4-(1E-pentenyl)cyclohexyloxycarbonyl]phenylester,
4-Octyloxybenzoesäure-4-[trans-4-(4-pentenyl)cyclohexyloxycarbonyl]phenylester,
4-Octyloxybenzoesäure-4-[trans-4-(5-hexenyl)cyclohexyloxycarbonyl]phenylester.

### Mischungsbeispiel A

27,2 Gew.-% 4-Dodecyloxybenzoesäure-4-[2-(trans-4-pentylcyclohexyl)äthyl]phenylester,
23,6 Gew.-% 4-(10-Undecenyloxy)benzoesäure-4-[2-(trans-4- pentylcyclohexyl)äthyl]phenylester,
17,0 Gew.-% 5-Decyl-2-(4-hexyloxyphenyl)pyrimidin,
17,0 Gew.-% 5-Decyl-2-(4-octyloxyphenyl)pyrimidin,
15,2 Gew.-% 5-Decyl-2-(4-decyloxyphenyl)pyrimidin,
Klp. (N-I) 100°C, Umwandlung N-S_{A} 80°C, Umwandlung S_{A}-S_{C} 69°C. Die S_{C}-Phase kristallisierte nicht beim Abkühlen bis -2°C.

### Mischungsbeispiel B

55 Gew.-% (S)-4-(6-Methyloctyloxy)benzoesäure-4-[2- (trans-4-pentylcyclohexyl)äthyl]phenylester,
45 Gew.-% (S)-4-Decyloxybenzoesäure-4-(2-methylbutyloxy)phenylester,
Klp. (Ch-I) 96,5°C, Umwandlung Ch-S*_{C} 64°C, Umwandlung S*_{C}-S 27°C.

### Mischungsbeispiel C

50 Gew.-% 4-Nonyloxybenzoesäure-4-[2-(trans-4-pentylcyclohexyl)äthyl]phenylester,
50 Gew.-% 4-Dodecyloxybenzoesäure-2-cyano-4-[2-(trans-4-pentylcyclohexyl)äthyl]phenylester,
Klp. (N-I) 134°C, Umwandlung N-S_{C} 68°C, Smp. (C-S_{C}) 46°C.

## Patentansprüche

1. Verbindungen der allgemeinen Formel 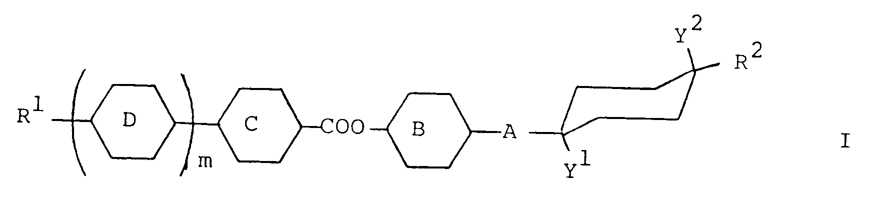 worin m für die Zahl 0 oder 1 steht; A eine einfache Kovalenzbindung, -CH₂-CH₂ -, -OCH₂-, -COO- oder -OOC- bedeutet; die Ringe B, C und D gegebenenfalls mit Cyano, Halogen oder Niederalkyl substituiertes 1,4-Phenylen bezeichnen; Y¹ und Y² Wasserstoff bedeuten oder einer der Substituenten Y¹ und Y² auch Cyano bedeutet; und R¹ und R² unabhängig voneinander gegebenenfalls halogensubstituiertes C₁-C₁₈-Alkyl oder gegebenenfalls halogensubstituiertes C₂-C₁₈--Alkenyl darstellen, in welchen gegebenenfalls eine oder zwei nicht benachbarte CH₂-Gruppen durch Sauerstoff ersetzt sind; mit der Massgabe, dass mindestens einer der Reste R¹ und R² ein chirales Kohlenstoffatom und/oder eine C-C-Doppelbindung aufweist, wenn A für -COO- steht, und mindestens einer der Reste R¹ und R² eine C-C-Doppelbindung aufweist, wenn A für eine einfache Kovalenzbindung steht.

2. Verbindungen nach Anspruch 1, dadurch gekennzeichnet, dass R¹ und R² unabhängig voneinander C₁-C₁₈-Alkyl oder C₂-C₁₈-Alkenyl darstellen, in welchem gegebenenfalls eine oder zwei nicht benachbarte CH₂-Gruppen durch Sauerstoff ersetzt sind.

3. Optisch aktive Verbindungen nach Anspruch 1 oder 2, dadurch gekennzeichnet, dass mindestens einer der Reste R¹ und R² ein chirales Kohlenstoffatom aufweist.

4. Optisch aktive Verbindungen nach Anspruch 3, dadurch gekennzeichnet, dass R¹ ein chirales Kohlenstoffatom aufweist.

5. Optisch aktive Verbindungen nach Anspruch 3 oder 4, dadurch gekennzeichnet, dass der Rest mit chiralem Kohlenstoffatom eine Gruppe der allgemeinen Formel 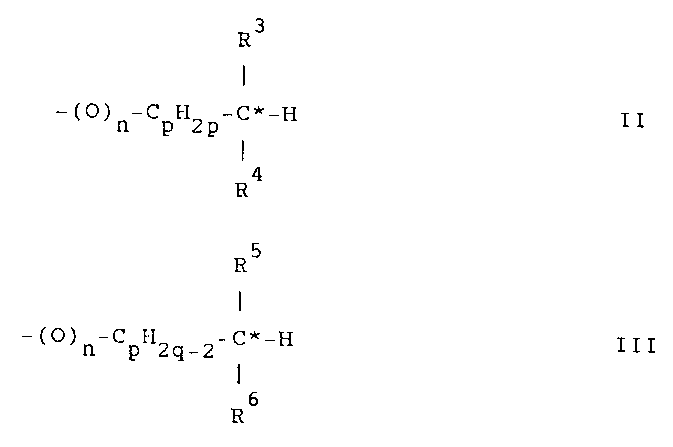 worin n, p und q ganze Zahlen bedeuten und n für 0 oder 1, p für 0-6 und q für 2-6 stehen; R³ Alkyl und R⁴ Halogen, Alkoxy, Alkenyl, Alkenyloxy oder von R³ verschiedenes Alkyl bedeuten; oder R³ Alkenyl und R⁴ Alkoxy bedeuten; R⁵ Alkyl und R⁶ Halogen, Alkoxy oder von R⁵ verschiedenes Alkyl bezeichnen; und C* das chirale Kohlenstoffatom bezeichnet,
bedeutet.

6. Optisch aktive Verbindungen nach Anspruch 5, dadurch gekennzeichnet, dass der Rest mit chiralem Kohlenstoffatom eine Gruppe der Formel II bedeutet, worin R³ Methyl und R⁴ von Methyl verschiedenes Alkyl bezeichnet.

7. Verbindungen nach einem der Ansprüche 1 bis 6, dadurch gekennzeichnet, dass A -CH₂CH₂-, -OCH₂ - oder -OOC-, vorzugsweise -CH₂CH₂- oder -OCH₂- bedeutet.

8. Verbindungen nach einem der Ansprüche 1 bis 7, dadurch gekennzeichnet, dass Y¹ und Y² Wasserstoff bedeuten und die Ringe C und D 1,4-Phenylen bezeichnen.

9. Verbindungen nach einem der Ansprüche 1 bis 8, dadurch gekennzeichnet, dass Ring B 1,4-Phenylen, 2-Cyano-1,4-phenylen, 2,3-Dicyano-1,4-phenylen, 2-Halogeno-1,4-phenylen oder 2,3-Dihalogeno-1,4-phenylen bezeichnet.

10. Verbindungen nach einem der Ansprüche 1 bis 9. dadurch gekennzeichnet, dass m für die Zahl 0 steht.

11. Verbindungen nach einem der Ansprüche 1 bis 10, dadurch gekennzeichnet, dass R¹ Alkoxy oder Alkenyloxy und R² Alkyl oder Alkenyl darstellen.

12. Verbindungen nach einem der Ansprüche 1 bis 11, dadurch gekennzeichnet, dass R¹ und R² zusammen mindestens 7, vorzugsweise mindestens 10 Kohlenstoffatome aufweisen.

13. Flüssigkristallines Gemisch mit mindestens 2 Komponenten, dadurch gekennzeichnet, dass mindestens eine Komponente eine Verbindung der in Anspruch 1 definierten Formel I ist.

14. Chiral smektisches Gemisch nach Anspruch 13, dadurch gekennzeichnet, dass es eine optisch aktive Verbindung der Formel I und/oder einen optisch aktiven Zusatz enthält.

15. Verfahren zur Herstellung der Verbindungen der in Anspruch 1 definierten Formel I, dadurch gekennzeichnet, dass man
a) eine Verbindung der allgemeinen Formel 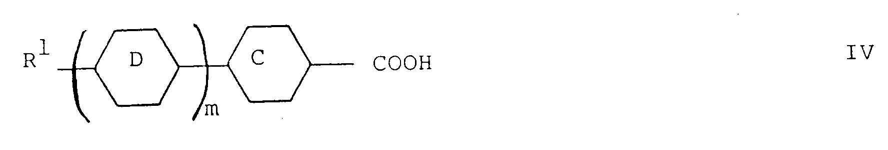 und eine Verbindung der allgemeinen Formel 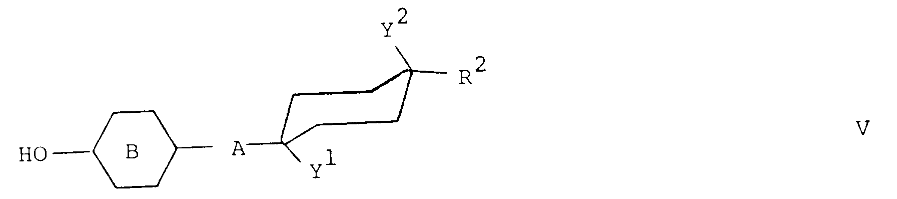 worin A, R¹, R², Y¹, Y², m und die Ringe B, C und D die in Anspruch 1 gegebenen Bedeutungen haben, oder reaktionsfähige Derivate dieser Verbindungen verestert, und dass man gewünschtenfalls eine erhaltene Verbindung der Formel I, worin Ring B, C oder D mit Chlor oder Brom substituiertes 1,4-Phenylen bedeutet, mit Kupfer-(I)-cyanid, Natriumcyanid oder Kaliumcyanid umsetzt, oder
b) zur Herstellung der Verbindungen der Formel I, worin A eine Estergruppe -COO- oder -OOC- bedeutet, eine Verbindung der allgemeinen Formel 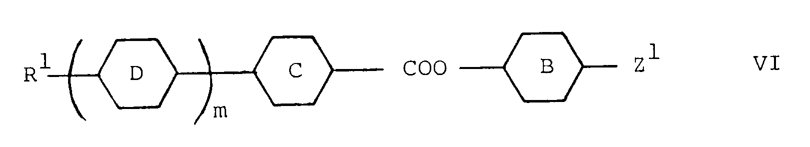 und eine Verbindung der allgemeinen Formel 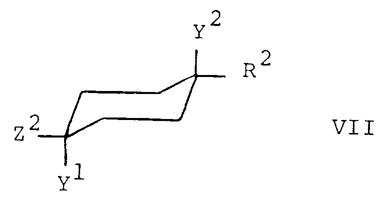 worin eine der Gruppen Z¹ und Z² die Carboxylgruppe und die andere die Hydroxygruppe bezeichnet, und R¹, R², Y¹, Y², m und die Ringe B, C und D die in Anspruch 1 gegebenen Bedeutungen haben,
oder reaktionsfähige Derivate dieser Verbindungen verestert, und dass man gewünschtenfalls eine erhaltene Verbindung der Formel I, worin Ring B, C oder D mit Chlor oder Brom substituiertes 1,4-Phenylen bedeutet, mit Kupfer-(I)-cyanid, Natriumcyanid oder Kaliumcyanid umsetzt.

16. Verwendung der Verbindungen der in Anspruch 1 definierten Formel I für elektro-optische Zwecke.

## Claims

1. Compounds of the general formula 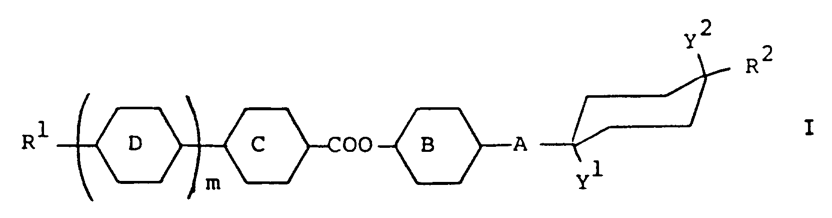 wherein m stands for the number 0 or 1; A signifies a single covalent bond, -CH₂-CH₂-, -OCH₂-, -COO- or -OOC-; rings B, C and D denote 1,4-phenylene optionally substituted with cyano, halogen or lower alkyl; Y¹ and Y² signify hydrogen or one of the substitutents Y¹ and Y² also signifies cyano; and R¹ and R² each individually represent optionally halogen-substituted C₁-C₁₈-alkyl or optionally halogen-substituted C₂-C₁₈-alkenyl in which optionally one CH₂ group or two non-adjacent CH₂ groups is/are replaced by oxygen; with the proviso that at least one of the residues R¹ and R² has a chiral carbon atom and/or a C-C double bond when A stands for -COO- and that at least one of the residues R¹ and R² has a C-C double bond when A stands for a single covalent bond.

2. Compounds according to claim 1, characterized in that R¹ and R² each independently represent C₁-C₁₈-alkyl or C₂-C₁₈-alkenyl in which optionally one CH₂ group or two non-adjacent CH₂ groups is/are replaced by oxygen.

3. Optically active compounds according to claim 1 or 2, characterized in that at least one of the residues R¹ and R² has a chiral carbon atom.

4. Optically active compounds according to claim 3, characterized in that R¹ has a chiral carbon atom.

5. Optically active compounds according to claim 3 or 4, characterized in that the residue with a chiral carbon atom signifies a group of the general formula 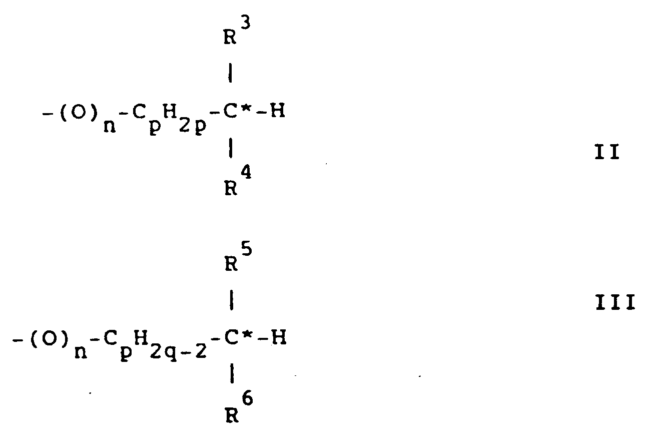 wherein n, p and q signify whole numbers and n stands for 0 or 1, p stands for 0-6 and q stands for 2-6; R³ signifies alkyl and R⁴ signifies halogen, alkoxy, alkenyl, alkenyloxy or alkyl different from R³; or R³ signifies alkenyl and R⁴ signifies alkoxy; R⁵ denotes alkyl and R⁶ denotes halogen, alkoxy or alkyl different from R⁵; and C* signifies the chiral carbon atom.

6. Optically active compounds according to claim 5, characterized in that the residue with a chiral carbon atom signifies a group of formula II in which R³ denotes methyl and R⁴ denotes alkyl different from methyl.

7. Compounds according to any one of claims 1 to 6, characterized in that A signifies -CH₂CH₂-, -OCH₂- or -OOC-, preferably -CH₂CH₂- or -OCH₂-.

8. Compounds according to any one of claims 1 to 7, characterized in that Y¹ and Y² signify hydrogen and rings C and D denote 1,4-phenylene.

9. Compounds according to any one of claims 1 to 8, characterized in that ring B denotes 1,4-phenylene, 2-cyano-1,4-phenylene, 2,3-dicyano-1,4-phenylene, 2-halogeno-1,4-phenylene or 2,3-dihalogeno-1,4-phenylene.

10. Compounds according to any one of claims 1 to 9, characterized in that m stands for the number O.

11. Compounds according to any one of claims 1 to 10, characterized in that R¹ represents alkoxy or alkenyloxy and R² represents alkyl or alkenyl.

12. Compounds according to any one of claims 1 to 11, characterized in that R¹ and R² together have at least 7, preferably at least 10, carbon atoms.

13. A liquid crystalline mixture with at least 2 components, characterized in that at least one component is a compound of formula I defined in claim 1.

14. A chiral smectic mixture according to claim 13, characterized in that it contains an optically active compound of formula I and/or an optically active additive.

15. A process for the manufacture of the compounds of formula I defined in claim 1, characterized by
a) esterifying a compound of the general formula 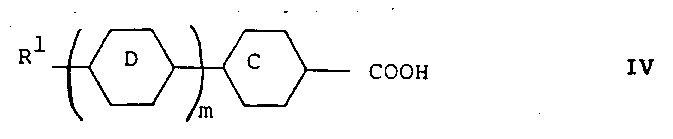 and a compound of the general formula 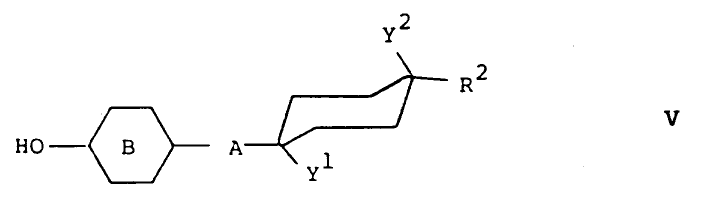 wherein A, R¹, R², Y¹, Y², m and rings B, C and D have the significances given in claim 1, or reactive derivatives of these compounds and, if desired, reacting a compound of formula I in which ring B, C or D signifies 1,4-phenylene substituted with chlorine or bromine with copper(I) cyanide, sodium cyanide or potassium cyanide, or
b) for the manufacture of compounds of formula I in which A signifies an ester group -COO- or -OOC-, esterifying a compound of the general formula 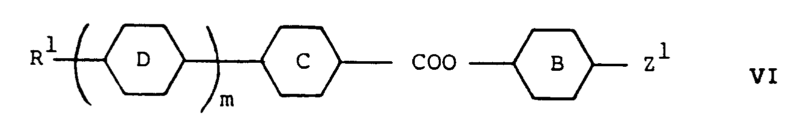 and a compound of the general formula 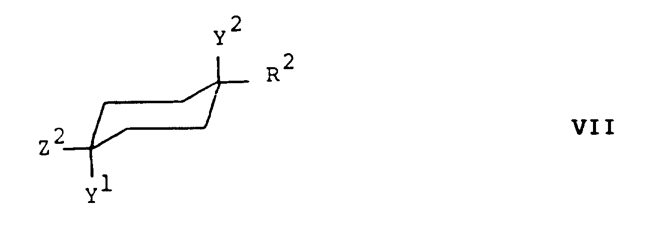 wherein one of the groups Z¹ and Z² denotes the carboxyl group and the other denotes the hydroxy group and R¹, R², Y¹, Y², m and rings B, C and D have the significances given in claim 1,
or reactive derivatives of these compounds and, if desired, reacting a compound of formula I obtained in which ring B, C or D signifies 1,4-phenylene substituted with chlorine or bromine with copper(I) cyanide, sodium cyanide or potassium cyanide.

16. The use of the compounds of formula I defined in claim 1 for electro-optical purposes.

## Revendications

1. Composés de formule générale 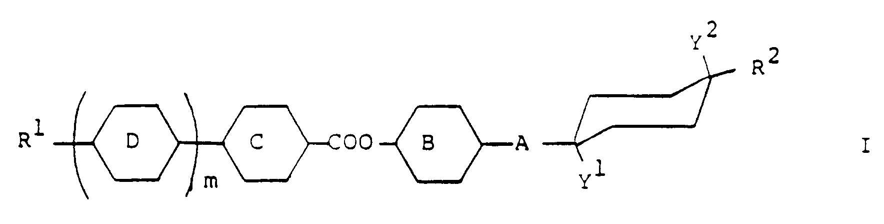 dans laquelle m vaut le nombre 0 ou 1 ; A représente une liaison de covalence simple ; représente -CH₂-CH₂-, -OCH₂-, -COO-, ou -OOC- ; les noyaux B, C et D représentent un 1,4-phénylène éventuellement substitué par un cyano, un halogène ou un alcoyle inférieur ; Y¹ et Y² représentent un hydrogène, ou l'un des substituants Y¹ et Y² représente également un cyano ; et R¹ et R² représentent indépendamment l'un de l'autre un alcoyle en C₁-₁₈ éventuellement halogéno-substitué, ou un alcényle en C₂-₁₈ éventuellement halogénésubstitué, dans lequel le cas échéant un ou deux groupes CH₂ non-voisins sont remplacés par l'oxygène ; sous réserve qu'au moins l'un des radicaux R¹ et R² présente un atome de carbone chiral et/ou une double liaison C=C lorsque A représente -COO-, et qu'au moins l'un des radicaux R¹ et R² représente une double liaison C=C lorsque A représente une liaison de covalence simple.

2. Composés selon la revendication 1, caractérisés en ce que R¹ et R² représentent indépendamment l'un de l'autre un alcoyle en C₁-₁₈ ou un alcényle en C₂-₁₈, dans lequel le cas échéant un ou deux groupes CH₂ non-voisins sont remplacés par un oxygène.

3. Composés optiquement actifs selon la revendication 1 ou 2, caractérisés en ce qu'au moins l'un des radicaux R¹ et R² présente un atome de carbone chiral.

4. Composés optiquement actifs selon la revendication 3, caractérisés en ce que R¹ présente un atome de carbone chiral.

5. Composés optiquement actifs selon la revendication 3 ou 4, caractérisés en ce que le radical présentant un atome de carbone chiral représente un groupe de formule générale 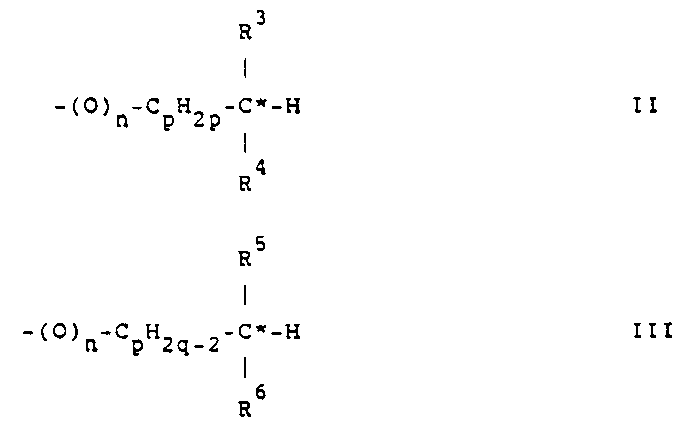 dans laquelle n, p et q représentent des nombres entiers et n vaut 0 ou 1, p vaut 0-6 et q vaut 2-6 ; R³ représente un alcoyle et R⁴ un halogène, un alcoxy, un alcényle, un alcynyloxy ou un alcoyle différent de R³ ; ou R³ représente un alcényle et R⁴ un alcoxy ; R⁵ un alcoyle et R⁶ un halogène, un alcoxy ou un alcoyle différent de R⁵ ; et C* représente l'atome de carbone chiral.

6. Composés optiquement actifs selon la revendication 5, caractérisés en ce que le radical ayant un atome de carbone chiral représente un groupe de formule II où R³ représente un méthyle et R⁴ est un alcoyle différent d'un méthyle.

7. Composés selon l'une quelconque des revendications 1 à 6, caractérisés en ce que A représente -CH₂CH₂-, -OCH₂- ou -OOC-, de préférence - CH₂CH₂- ou -OCH₂.

8. Composés selon l'une des revendications 1 à 7, caractérisés en ce qu'Y¹ et Y² représentent un hydrogène et les noyaux C et D représentent un 1,4-phénylène.

9. Composés selon l'une des revendications 1 à 8, caractérisés en ce que le noyau B représente un 1,4-phénylène, le 2-cyano-1,4-phénylène, un 2,3-dicyano-1,4-phénylène, un 2-halogéno-1,4-phénylène, ou un 2,3-dihalogéno-1,4-phénylène.

10. Composés selon l'une des revendications 1 à 9, caractérisés en ce que m vaut le nombre 0.

11. Composés selon l'une des revendications 1 à 10, caractérisés en ce que R¹ représente un alcoxy ou un alcényloxy et R² un alcoyle ou un alcényle.

12. Composés selon l'une des revendications 1 à 11, caractérisés en ce que R¹ et R² représentent ensemble au moins 7, de préférence au moins 10 atomes de carbone.

13. Mélange à cristaux liquides à au moins deux composants, caractérisé en ce qu'au moins un composant est un composé de formule I définie dans la revendication 1.

14. Mélange smectique chiral selon la revendication 13, caractérisé en ce qu'il contient un composé optiquement actif de formule I et/ou un additif optiquement actif.

15. Procédé de préparation des composés de formule I définis dans la revendication 1, caractérisé en ce que
(a) on estérifie un composé de formule générale 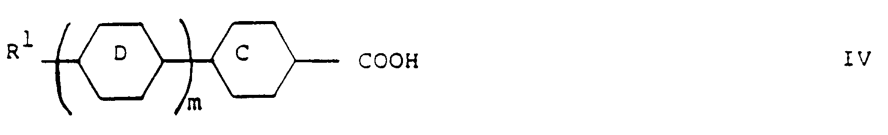 et un composé de formule générale 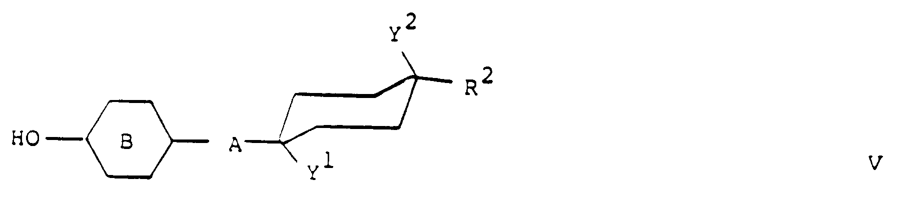 dans laquelle A, R¹, R, Y¹, Y², m et les noyaux B, C et D ont la signification donnée dans la revendication 1,
ou des dérivés réactifs de ces composés, et en ce que, si on le désire, on fait réagir un composé obtenu de formule I, où les noyaux B, C ou D représentent un 1,4-phénylène substitué par un chlore ou un brome, avec du cyanure de cuivre (I), du cyanure de sodium ou du cyanure de potassium, ou
b) pour préparer des composés de formule I dans lesquels A représente un groupe ester -COO- ou -OOC-, on estérifie un composé de formule générale 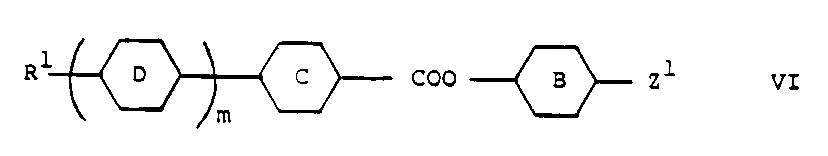 et un composé de formule 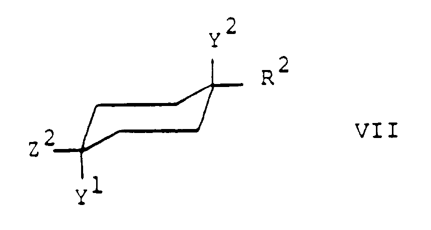 générale dans laquelle l'un des groupes Z¹ et Z² représente le groupe carboxyle et l'autre le groupe hydroxy, et R¹, R², Y¹, Y², m et les noyaux B, C et D ont la signification donnée dans la revendication 1,
ou des dérivés réactifs de ces composés, et en ce que, si on le désire, on fait réagir un composé obtenu de formule I, où les noyaux B, C ou D représentent un 1,4-phénylène substitué par du chlore ou du brome, avec du cyanure de cuivre (I), du cyanure de sodium ou du cyanure de potassium.

16. Application des composés de formule I définis dans la revendication 1 dans des buts électro-optiques.
